# EUROPEAN PATENT APPLICATION

(11) **EP 1 939 612 A1**
(43) Date of publication of application: **02.07.2008**
(21) Application number: 06811833.0
(22) Date of filing: 16.10.2006
(51) Int. Cl.: G01N 27/327

(54) **BIOSENSOR CHIP AND PROCESS FOR PRODUCING THE SAME**

(30) Priority: 17.10.2005 JP 2005302330
(71) Applicant: Sumitomo Electric Industries, Ltd., Osaka-shi, Osaka 541-0041 (JP); National Institute of Advanced Industrial Science and Technology, Tokyo 100-8921 (JP)
(72) Inventor: KITAMURA, Takahiko, Oaka-shi, Osaka 5540024 (JP); HOSOYA, Toshifumi, Osaka-shi, Osaka 5540024 (JP); KAIMORI, Shingo, Osaka-shi, Osaka 5540024 (JP); ICHINO, Moriyasu, Osaka-shi, Osaka 5540024 (JP); NAKAMURA, Hideaki, Tsukuba-shi, Ibaraki 3058562 (JP); KARUBE, Isao, Tsukuba-shi, Ibaraki 058562 (JP); GOTOH, Masao, Tsukuba-shi, Ibaraki 3058562 (JP); ISHIKAWA, Tomoko, Tsukuba-shi, Ibaraki 3058562 (JP)
(74) Representative: Cross, Rupert Edward Blount
(86) International application number: PCT/JP2006/320569
(87) International publication number: WO 2007/046334

(57) **Abstract**

It is intended to provide a biosensor chip capable of rapid and correct measurement, including a reaction chamber that enables a measurement of a very small amount of a measurement sample and has a small capacity and potassium ferricyanide having a very small crystal particle size disposed in the reaction chamber.

In a biosensor chip 1, electrodes 3 and 4 are disposed on the lower substrate 2, and lower spacer 5 (7 and 8) is adhered on the electrodes 3 and 4. An upper spacer 11 (12 and 13) is adhered to an upper substrate 15, and a lower spacer 5 is attached to the upper spacer 11 by and adhesive agent 10. A lower groove 9 is formed between long and short lower spacers 7 and 8, and an upper groove 14 is formed between long and short spacers 12 and 13, so that a reaction chamber 17 is formed by the upper and lower grooves 9 and 14. A capacity of the reaction chamber is 0.3 µL, and an enzyme 18 and potassium ferricyanide are disposed in the reaction chamber 17 in such a fashion as to oppose to each other with a gap being defined therebetween. A crystal particle diameter of the potassium ferricyanide is 100 µm or less, and amount of the potassium ferricyanide is V × 0.1 mg or more when the capacity of the reaction chamber 17 is V.

## Description

### Technical Field

This invention relates to a biosensor chip for performing a biochemical reaction using a very small amount of a sample to be measured.

### Related Art

A biosensor chip is a sensor chip that causes a biochemical reaction such as an enzyme reaction and an antigen-antibody reaction on a very small amount of a sample introduced into a reaction chamber thereof and then outputs information obtained by the biochemical reaction via an electrode. Such biosensor chip utilizes the excellent molecular discrimination function of living body and enables a rapid and convenient measurement of a very small amount of a chemical substance. For example, the biosensor chip is used as a blood sugar level sensor or as a urinary sugar level sensor for measuring a glucose amount (blood sugar level) in blood or a urinary sugar level for an in-home medical checkup (self-medical cares) for self-managing and preventing diabetes.

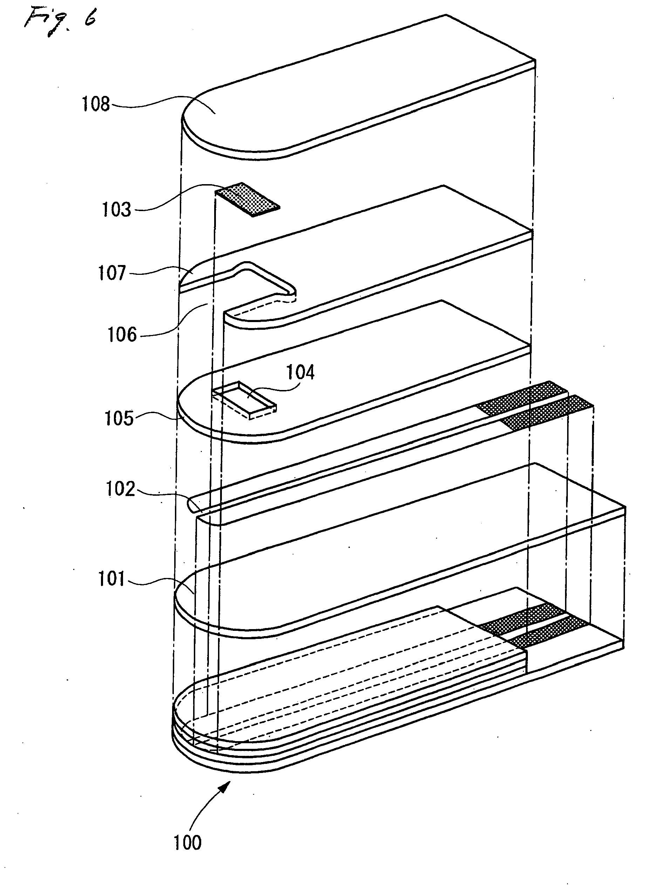

As one example of conventional biosensor chips, the one disclosed in Patent Publication 1 is known. This biosensor chip, as an enzyme sensor 100, is provided with an electrode unit 102 formed on an electrically insulating substrate 101 and including two electrodes in the form of stripes as shown in Fig. 6. A reaction layer 103 is closely fixed to one end portion of the electrode unit 102, and potassium ferricyanide is contained in the reaction layer 103 as one example of an electron mediator. A mask layer 105 having a window 104 is disposed above the electrode unit 102; a spacer 107 having a test liquid inlet 106 is disposed above the mask layer 105; and a protection layer 108 is disposed above the spacer 107. Therefore, the enzyme sensor 100 is formed of the electrically insulating substrate 101, the electrode unit 102, the mask layer 105, the spacer 107, and the protection layer 108 that are stacked.

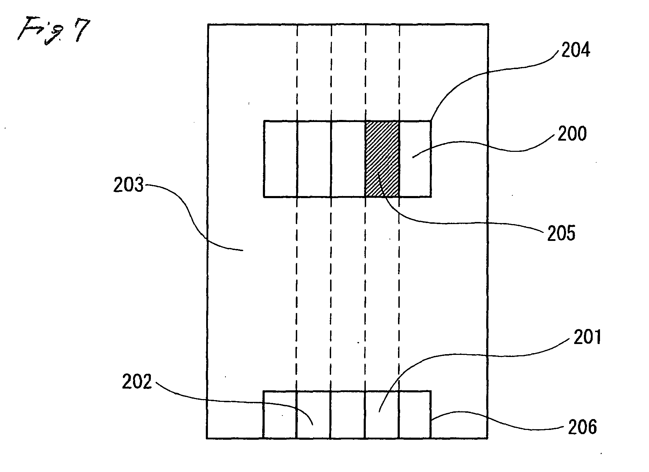

As another example of the conventional biosensor chips, the one disclosed in Patent Publication 2 is known. In this biosensor strip as shown in Fig. 7, a support electrode 201 and a standard reference electrode 202 are disposed on a first electrode insulator 200, and a second electrical insulator 203 is disposed on the electrodes. In this biosensor strip, a notched portion 204 is formed, and a reagent 205 is placed on the support electrode 201 exposed in the notched portion 204. The reagent 205 contains an enzyme and potassium ferricyanide and a reagent prepared in the form of a liquid is dried on a surface of a support electrode 201 in the notched portion 204. An additional notched portion 206 is included for the purpose of facilitating electrical connection between the support electrode 201 and the standard reference electrode 202 and a potential difference meter.

Patent Publication 1: JP-A-2001-311712
Patent Publication 2: JP-T-9-500727

### Disclosure of the Invention

### Problems to be solved by the Invention

Recently, there is a demand for a biosensor chip in which a capacity of a reaction chamber for mixing and reacting an enzyme or an electron mediator with a measurement sample is downsized. For example, in the case of measuring a blood sugar level by using a blood of a subject as a measurement sample, it is possible to realize the blood sugar level measurement by drawing a very small amount of the blood, thereby diminishing a blood draw load on the subject. In the case where potassium ferricyanide is used as the electron mediator in the downsized reaction chamber of the biosensor chip, a crystal particle size of potassium ferricyanide presents a problem. When a mixture solution containing potassium ferricyanide is coated and dried inside the reaction chamber of the biosensor chip, the crystal particle size can be large since potassium ferricyanide is easily crystallized. When a blood is introduced into the reaction chamber in the biosensor chip accommodating the potassium ferricyanide having the large crystal particle size, it is sometime impossible to perform a correct measurement since such potassium ferricyanide is not dissolved rapidly. Also, in the case where potassium ferricyanide in which crystal particles having a large size and crystal particle having a small size are mixed is placed in the reaction chamber of the biosensor chip, a measurement value can be fluctuated due to a fluctuation in dissolved state of potassium ferricyanide. Further, when potassium ferricyanide in which crystal particles having a large size and crystal particle having a small size are mixed is gathered at an inlet of the reaction chamber, it is considered that it is difficult to introduce the blood used as the measurement reagent into the reaction chamber.

An object of this invention is to provide a biosensor chip capable of a rapid and correct measurement, comprising a reaction chamber of a small capacity that enables a measurement of a very small amount of a measurement sample and potassium ferricyanide that has a very small crystal particle size and is disposed in the reaction chamber.

### Means for solving the Problems

According to this invention, there is provided a biosensor chip including: upper and lower substrates, at least two electrodes disposed on at least one of the upper and lower substrates, and a reaction chamber for performing a chemical reaction, wherein at least an enzyme and potassium ferricyanide are included as reagents to be placed in the reaction chamber; a capacity of the potassium ferricyanide placed in the reaction chamber is V × 0.1 mg or more when a capacity of the reaction chamber is V µL; and a maximum diameter of crystal particles of the potassium ferricyanide is 100 µm.

Also, in the biosensor chip according to this invention, it is preferable that the maximum diameter of the crystal particles of the potassium ferricyanide is 50 µm or less.

Also, in the biosensor chip according to this invention, it is preferable that the enzyme and the potassium ferricyanide are placed in the reaction chamber with a gap being defined therebetween.

Also, in the biosensor chip according to this invention, it is preferable that the upper and lower substrates are formed from one sheet, and the one sheet is folded to form the upper and lower substrates.

According to this invention, there is provided a biosensor chip production method for producing a biosensor chip including upper and lower substrates, at least two electrodes disposed on at least one of the upper and lower substrates, and a reaction chamber for performing a chemical reaction, the method including: a step of coating at least an enzyme and potassium ferricyanide in the reaction chamber, and a step of freezing, heating, or mixing with a poor solvent the potassium ferricyanide in such a manner that a maximum diameter of crystal particles of the potassium ferricyanide becomes 100 µm or less and a capacity of the potassium ferricyanide placed in the reaction chamber becomes V × 0.1 mg or more when a capacity of the reaction chamber is V µL.

Further, in the biosensor production method according to this invention, it is preferable that one sheet is folded to form the upper and lower substrates.

### Effect of the Invention

According to the biosensor chip and the biosensor production method according to this invention, since the potassium ferricyanide of very fine crystal particles is placed in the reaction chamber, the potassium ferricyanide is rapidly and uniformly dissolved by using a very small amount of a measurement sample, thereby enabling a correction measurement wherein a measurement result fluctuation is suppressed.

### Brief Description of the Drawings

[Fig. 1]

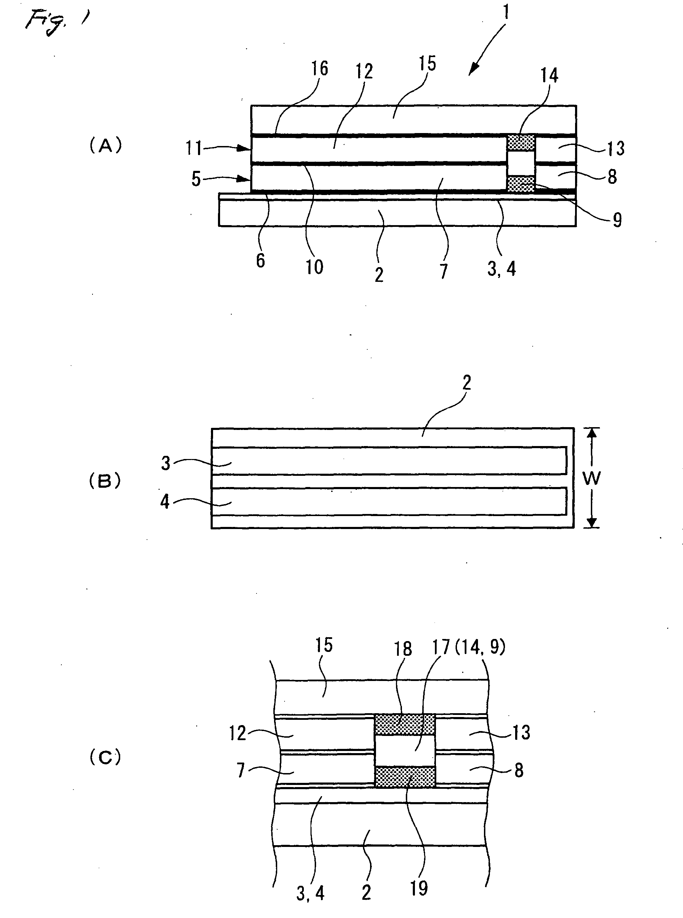

   Fig. 1 shows a biosensor chip according to this invention, wherein (A) is a diagram showing the biosensor chip from a lateral direction; (B) i.s a diagram showing a lower substrate and an electrode; and (C) is an enlarged view of a reaction chamber.
[Fig. 2]

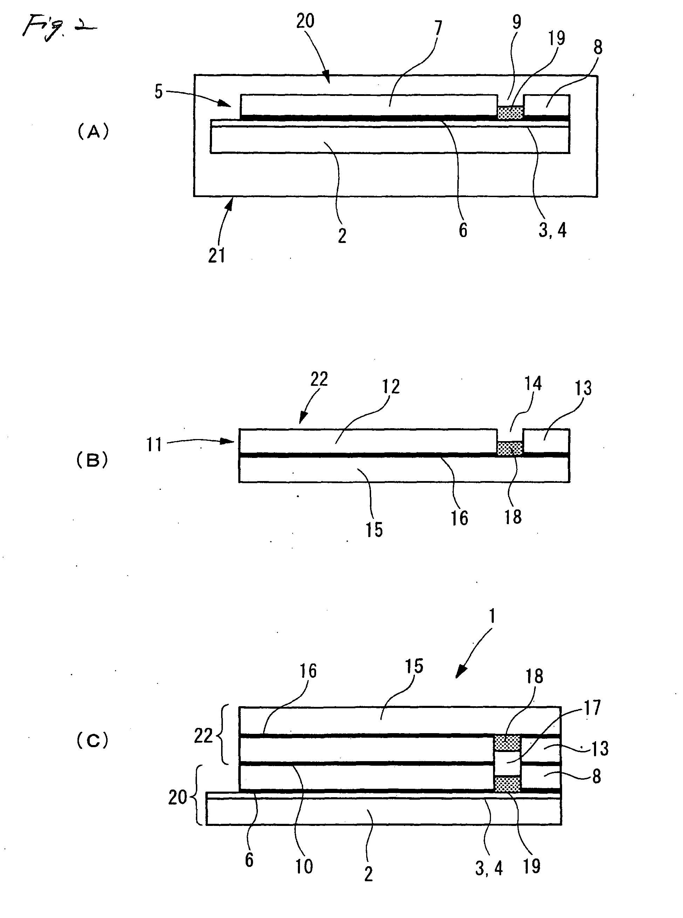

   Fig. 2 shows diagrams for illustrating a biosensor chip production method, wherein (A) is a diagram for illustrating a lower part; (B) is a diagram for illustrating an upper part; and (C) is a diagram for illustrating the upper part and the lower part that are attached to each other.
[Fig. 3]

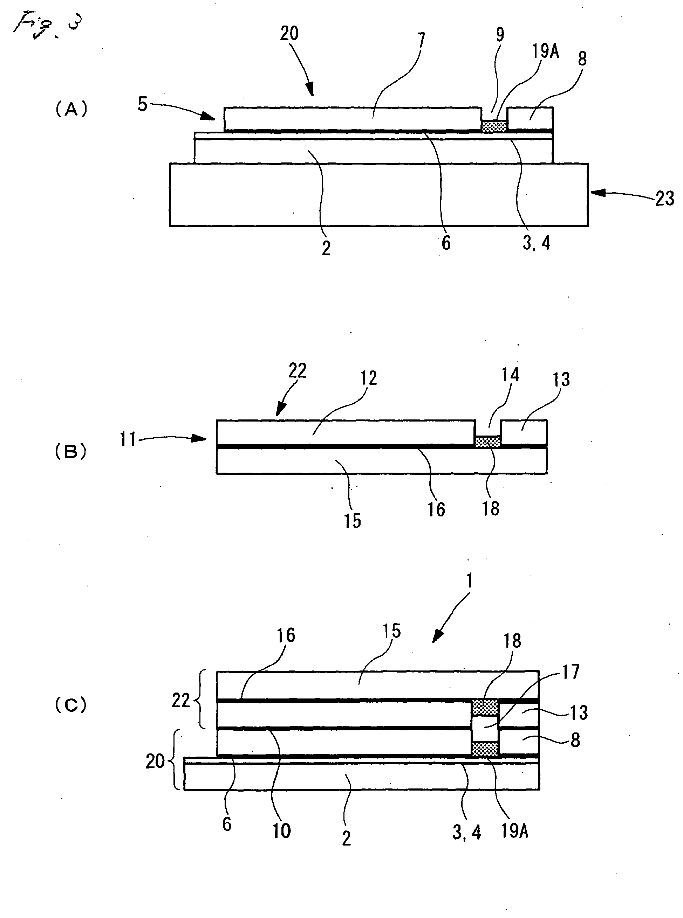

   Fig. 3 shows diagrams for illustrating a biosensor chip production method, wherein (A) is a diagram for illustrating a lower part; (B) is a diagram for illustrating an upper part; and (C) is a diagram for illustrating the upper part and the lower part that are attached to each other.
[Fig. 4]

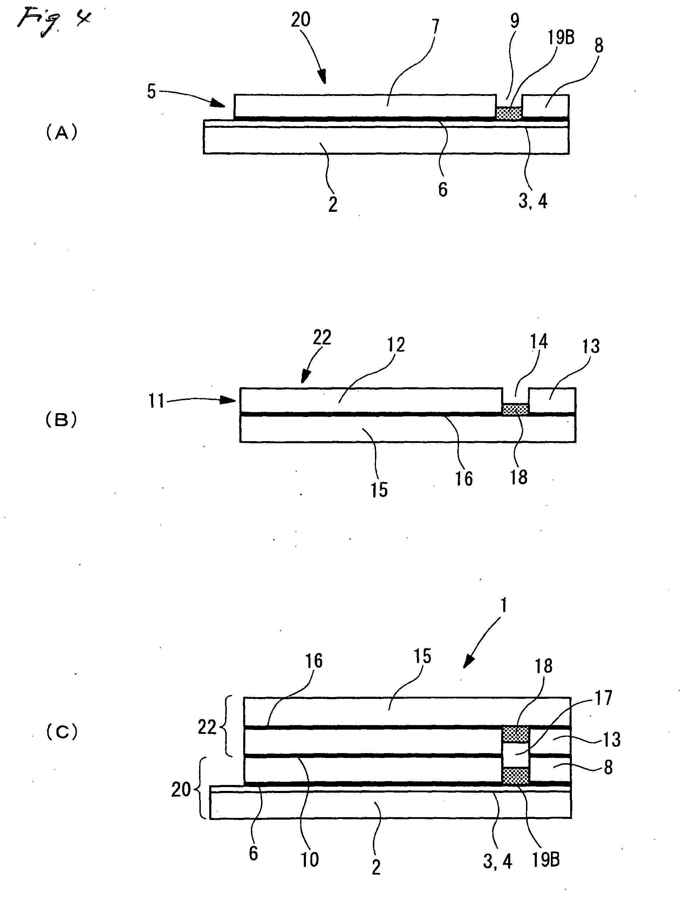

   Fig. 4 shows diagrams for illustrating a biosensor chip production method, wherein (A) is a diagram for illustrating a lower part; (B) is a diagram for illustrating an upper part; and (C) is a diagram for illustrating the upper part and the lower part that are attached to each other.
[Fig. 5]

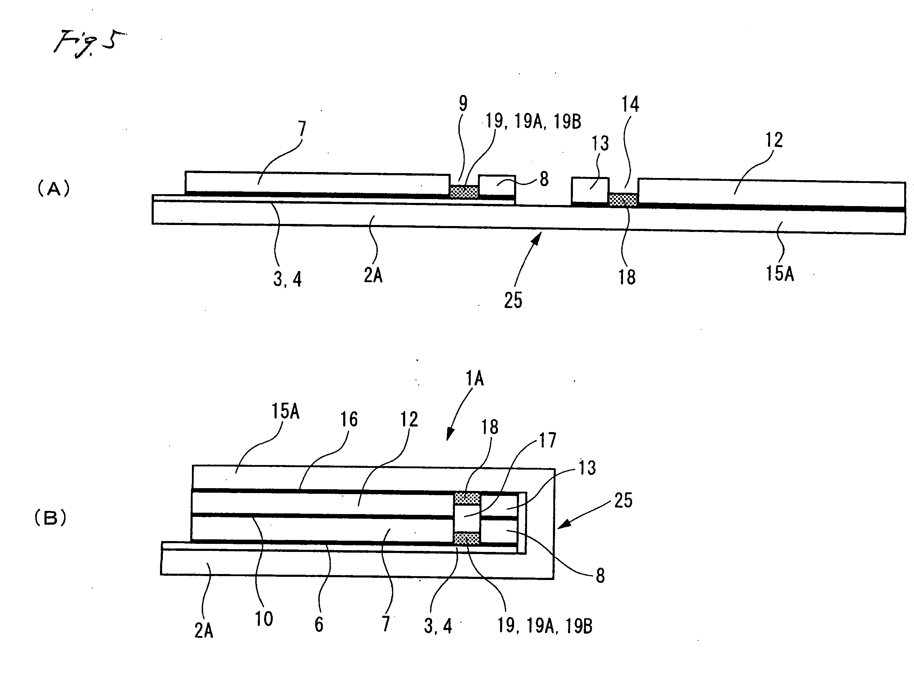

   Fig. 5 shows for illustrating a biosensor chip production method using one insulating substrate sheet, wherein (A) is a diagram for illustrating a state before folding the sheet, and (B) is a diagram for illustrating a state after folding the sheet.
[Fig. 6]
   Fig. 6 is a perspective view showing one example of conventional biosensor chip.
[Fig. 7]
   Fig. 7 is a diagram showing another example of conventional biosensor chip.

### Description of Reference Numerals and Signs

1: biosensor
2: lower substrate
3, 4: electrode
5: lower spacer
6: adhesive agent
7: long lower spacer
8: short lower spacer
9: lower groove
10: adhesive agent
11: upper spacer
12: long upper spacer
13: short upper spacer
14: upper groove
15: upper substrate
16: adhesive agent
17: reaction chamber
18: enzyme
19, 19A, 19B: potassium ferricyanide

### Best Mode for Carrying out the Invention

Hereinafter, a biosensor chip and a biosensor chip production method according to this invention will be described in detail with reference to the drawings.

Fig. 1 shows one example of embodiments of the biosensor chip according to this invention. As shown in Fig. 1(A), a biosensor chip 1 has an insulating lower substrate 2, and two electrodes 3 and 4 are disposed on an upper surface of the lower substrate 2 in parallel to each other with a gap being defined therebetween (see Fig. 1(B)). A lower spacer 5 is fixed on the electrodes 3 and 4 with an adhesive agent 6. The lower spacer 5 has a width W that is substantially the same as that of the lower substrate 2 and includes a long lower spacer 7 and a short lower spacer disposed with a gap being defined therebetween, and a lower groove 9 is formed between the long lower spacer and the short lower spacer.

An upper spacer 11 is fixed on the lower spacer 5 with an adhesive agent 10. The upper spacer 11 has a size and a width that are the same as those of the lower spacer 5 and includes a long upper spacer 12 and a short upper spacer 13 disposed with a gap being defined therebetween, and an upper groove 14 is formed between the long upper spacer and the short upper spacer. An upper substrate 15 is' fixed on the upper spacer 11 with an adhesive agent 16. Therefore, this biosensor chip 1 has a structure that the lower substrate 2, the electrodes 3 and 4, the lower spacer 5, the upper spacer 11, and the upper substrate 15 are stacked. Though the lower substrate 2 and the upper substrate 15 are formed as separate members in this embodiment, the lower substrate 2 and the upper substrate 15 may be formed integrally. That is, it is possible to form a lower substrata and an upper substrate by folding one sheet into a laterally-faced U-shape.

A space enclosed by the upper and lower substrates 2 and 15, the upper and lower long spacers 7 and 12, and the upper and lower short spacers 8 and 13 serves as a reaction chamber 17 as shown in Fig. 1(C). The upper groove 9 and the lower groove 14 are opposed to each other to form the reaction chamber 17, and a capacity of the reaction chamber 17 is set to 0.3 µL or less. For example, when the capacity of the reaction chamber 17 is set to 0.3 µL, a capacity of the lower groove 9 can be set to 0.15 µL, and a capacity of the upper groove 14 can be set to 0.15 µL, so that each of the lower groove 9 and the upper groove 14 has the capacity that is half of that of' the reaction chamber 17. The capacity of the reaction chamber may be 0.3 µL or less, preferably 0.2 to 0.3 µL. Since an amount of the measurement reagent is very small when the capacity of the reaction chamber is 0.3 µL or less, it is possible to perform collection of the measurement reagent easily. When the capacity of the reaction chamber is more than 0.3 µL, a collection load of the measurement reagent, such as a blood draw load in the case of a blood sugar level measurement, is increased.

An enzyme 18 is coated on the upper substrate 15 in the reaction chamber 17, so that a biochemical reaction such as an enzyme reaction and an antigen-antibody reaction is caused when the measurement reagent flows into the reaction chamber 17. Potassium ferricyanide 19 serving as an electron mediator is coated on the lower substrate 2 and the electrodes 3 and 4 in the reaction chamber 17, and the potassium ferricyanide 19 is disposed with a gap being defined between the potassium ferricyanide 19 and the enzyme 18. Since the enzyme 18 and the potassium ferricyanide 19 are not mixed, it is possible to maintain activity of the enzyme 18 for a long period of time. Since the capacity of the reaction chamber 17 is very small (0.3 µL), it is important to maintain a high enzyme activity in order to obtain a correct measurement result by using the very small amount of measurement reagent.

In order to obtain the correct measurement result, a capacity of the potassium ferricyanide is also important, and it is necessary to keep the capacity of the potassium ferricyanide 1.9 to V × 0.1 mg or more when the capacity of the reaction chamber 17 is V. For example, when the capacity of the reaction chamber is 0.3 µL, a required amount of the potassium ferricyanide is 0.03 mg or more. When the amount of potassium ferricyanide is V × 0.1 mg or more, the potassium ferricyanide reacts with the very small amount of measurement reagent satisfactorily to give a correct measurement result.

Further, in the reaction chamber having the very small capacity of 0.3 µL or less, a dissolved state of the potassium ferricyanide influences on the measurement results when the very small amount of measurement reagent is introduced. In the biosensor chip according to this invention, it is necessary that a maximum diameter of crystal particles of the potassium ferricyanide is 100 µm or less in order to dissolve the potassium ferricyanide rapidly and uniformly. When the maximum diameter of very fine crystal particles of the potassium ferricyanide is 100 µm or less, the potassium ferricyanide can be dissolved rapidly and uniformly in the reaction chamber of 0.3 µL or less, thereby obtaining a correct measurement.

When the maximum diameter of the crystal particles of the potassium ferricyanide is 100 µm or more, it is difficult to rapidly dissolve the potassium ferricyanide, thereby causing a fluctuation in measurement results and a long reaction time. Also, in the case where crystal particles having a large size and crystal particle having a small size are mixed in the potassium ferricyanide, a fluctuation in states of dissolution of the potassium ferricyanide is caused to make it difficult to obtain correct measurement results. Therefore, since the potassium ferricyanide is dissolved remarkably rapidly and uniformly when the maximum diameter of the crystal particles of the potassium ferricyanide is 50 µm or less, the maximum diameter of 50 µm or less is more preferable. It is possible to obtain the potassium ferricyanide having the maximum diameter of 100 µm or less or 50 µm or less by coating an aqueous solution containing the potassium ferricyanide in the reaction chamber 17 and then performing freezing, heating, or mixing with a poor solvent.

Hereinafter, a biosensor chip production method according to this invention will be described. Fig. 2 shows one embodiment of biosensor chip production method according to this invention. The component parts that are the same as those of the biosensor chip of Fig. 1 are denoted by the same reference numerals, and detailed description of such component parts is omitted in the following description. Fig. 2 (A) shows a lower part 20 including the lower substrate 2 of the biosensor chip 1. The electrodes 3 and 4 are attached to the lower substrate 2 by screen printing or the like, and the spacer 5 is attached with the adhesive agent 6. The aqueous solution containing the potassium ferricyanide 19 is coated on the lower groove 9 formed by the long spacer 7 and the short spacer 8. After that, the lower part 20 of the biosensor chip 1 is disposed in a freezing apparatus 21 to freeze the aqueous solution containing the potassium ferricyanide 19. A freezing temperature may preferably be -20°C or less. When the potassium ferricyanide 19 is sufficiently frozen, the lower part 20 is taken out from the freezing apparatus 12 to be air-dried or to be vacuum-dried. By freezing and drying the aqueous solution containing the potassium ferricyanide 19 as described above, it is possible to precipitate fine crystal particles of the potassium ferricyanide. A maximum diameter of the crystal particles of the potassium ferricyanide 19 measured after the drying is 100 µm or less. Also, crystal particles of potassium ferricyanide that is quick-frozen by a freezing apparatus 21 had a maximum diameter of 50 µm or less.

An upper part 22 including the upper substrate 15 of the biosensor chip 1 is formed as shown in Fig. 2(B). The adhesive agent 16 is coated on the upper substrate 15 to attach the upper spacer 11. An aqueous solution containing the enzyme 18 is coated on the upper groove 14 formed by the long spacer 12 and the short spacer 13. Examples of the enzyme include glucose oxidase (GOD). After drying the aqueous solution containing the enzyme 18, the upper part 20 and the lower part 22 of the biosensor chip 1 are attached to each other with an adhesive agent 10 as shown in Fig. 2(C). The reaction chamber 17 is formed by the upper groove 9 and the lower groove 14 that are opposed to each other. In the reaction chamber 17, since the potassium ferricyanide 19 and the enzyme 18 are opposed to each other with a gap being defined therebetween, the enzyme 18 is not mixed with the potassium ferricyanide 19, and activity of the enzyme 18 is maintained. The reaction chamber 17 has a capacity of 0.3 µL or less and accommodates the potassium ferricyanide 19 in an amount of V × 0.1 mg or more when the capacity of the reaction chamber is V, and the maximum diameter of the crystal particles is 100 µm or less.

Fig. 3 shows another embodiment of biosensor chip production method according to this invention. The component parts that are the same as those of the biosensor chip of Fig. 1 are denoted by the same reference numerals, and detailed description of such component parts is omitted in the following description. Fig. 3(A) shows a lower part 20 including the lower substrate 2 of the biosensor chip 1. The electrodes 3 and 4 are attached to the lower substrate 2 by screen printing or the like, and the spacer 5 is attached with the adhesive agent 6. An aqueous solution containing a potassium ferricyanide 19A is coated on the lower groove 9 formed by the long spacer 7 and the short spacer 8. After that, a heating apparatus 23 is provided and the lower part 20 of the biosensor chip 1 including the lower substrate 2 is placed on a top face of the heating apparatus 23. After starting the heating device 23, the aqueous solution containing the potassium ferricyanide 19A is heated to evaporate moisture. When the moisture is sufficiently evaporated, the lower part 20 of the biosensor chip 1 is taken out from the heating apparatus 23 to be cooled. By heating the aqueous solution containing the potassium ferricyanide 19A as described above, it is possible to precipitate fine crystal particles of the potassium ferricyanide. A maximum diameter of the crystal particles of the potassium ferricyanide 19A measured after the cooling is 100 µm or less.

An upper part 22 including the upper substrate 15 of the biosensor chip 1 is formed as shown in Fig. 3 (B). The adhesive agent 16 is coated on the upper substrate 15 to attach the upper spacer 11. An aqueous solution containing the enzyme 18 is coated on the upper groove 14 formed by the long spacer 12 and the short spacer 13. Examples of the enzyme include glucose oxidase (GOD). After drying the aqueous solution containing the enzyme 18, the upper part 20 and the lower part 22 of the biosensor chip 1 are attached to each other with an adhesive agent 10 as shown in Fig. 3(C). The reaction chamber 17 is formed by the upper groove 9 and the lower groove 14 that are opposed to each other. In the reaction chamber 17, since the potassium ferricyanide 19A and the enzyme 18 are opposed to each other with a gap being defined therebetween, the enzyme 18 is not mixed with the potassium ferricyanide 19A, and activity of the enzyme 18 is maintained. The reaction chamber 17 has a capacity of 0.3 µL or less and accommodates the potassium ferricyanide 19A in an amount of V × 0.1 mg or more when the capacity of the reaction chamber is V, and the maximum diameter of the crystal particles is 100 µm or less.

Fig. 4 shows yet another embodiment of the biosensor chip production method according to this invention. The component parts that are the same as those of the biosensor chip of Fig. 1 are denoted by the same reference numerals, and detailed description of such component parts is omitted in the following description. Fig. 4(A) shows a lower part 20 including the lower substrate 2 of the biosensor chip 1. The electrodes 3 and 4 are attached to the lower substrate 2 by screen printing or the like, and the spacer 5 is attached with the adhesive agent 6. An aqueous solution containing a potassium ferricyanide 19B is coated on the lower groove 9 formed by the long spacer 7 and the short spacer 8. After that, ethanol which is a poor solvent for potassium ferricyanide is coated on the aqueous solution so that the aqueous solution is mixed with ethanol. Due to the presence of ethanol, the potassium ferricyanide is precipitated in the form of a microcrystal. Such method is known as a solvent reprecipitation method for precipitating microcrystal. In the case of using ethanol, the heating device used in the embodiment of Fig. 3 is not required, and it is possible to perform moisture evaporation at an ordinary temperature. Also, it is possible to use any of poor solvents of potassium ferricyanide insofar as the poor solvent dissolves well in water, and examples of such poor solvent include acetone. Fine crystal particles of the potassium ferricyanide 19B are precipitated by the mixing with ethanol. A maximum diameter of the crystal particles of the potassium ferricyanide 19B measured after the solvent evaporation is 100 µm or less. Also, when a mixing ratio of water and ethanol is set to 1:1 or more, fine crystal particles having a maximum diameter of 50 µm or less is precipitated, which is detected by measurement of crystal particles of the potassium ferricyanide 19B.

An upper part 22 including the upper substrate 15 of the biosensor chip 1 is formed as shown in Fig. 4 (B). The adhesive agent 16 is coated on the upper substrate 15 to attach the upper spacer 11. An aqueous solution containing the enzyme 18 is coated on the upper groove 14 formed by the long spacer 12 and the short spacer 13. Examples of the enzyme include glucose oxidase (GOD). After drying the aqueous solution containing the enzyme 18, the upper part 20 and the lower part 22 of the biosensor chip 1 are attached to each other with an adhesive agent 10 as shown in Fig. 4(C). The reaction chamber 17 is formed by the upper groove 9 and the lower groove 14 that are opposed to each other. In the reaction chamber 17, since the potassium ferricyanide 19B, and the enzyme 18 are opposed to each other with a gap being defined therebetween, the enzyme 18 is not mixed with the potassium ferricyanide 19B, and activity of the enzyme 18 is maintained. The reaction chamber 17 has a capacity of 0.3 µL or less and accommodates the potassium ferricyanide 19B in an amount of V × 0.1 mg or more when the capacity of the reaction chamber is V, and the maximum diameter of the crystal particles is 100 µm or less.

As described above, it is possible to precipitate fine crystal particles of the potassium ferricyanide by subjecting the potassium ferricyanide to the freezing, the heating, or the mixing with poor solvent in the biosensor chip production method according to this invention. Since it is possible to maintain the maximum diameter of the crystal particles of the potassium ferricyanide which is used as the electron mediator to 100 µm or less, preferably to 50 µm or less, it is possible to dissolve the potassium ferricyanide in the reaction chamber having the capacity of 0.3 µL rapidly and uniformly by using a very small amount of measurement reagent.

Though the examples in each of which the upper part including the upper substrate of the biosensor chip and the lower part including the lower substrate are attached to each other to produce the biosensor chip are described in the embodiments shown in Figs. 2 to 4, it is possible to produce a biosensor chip by using an integrated upper and lower substrate without using the separate upper substrate and the lower substrate in a biosensor chip production method according to this invention. Fig. 5 shows one example of producing a biosensor chip by using one sheet substrate. As shown in Fig. 5(A), this biosensor chip 1A has one insulating sheet substrate 25, and a left part of the sheet substrate 25 serves as a lower substrate 2A. On the lower substrate 2A, two electrodes 3 and 4 are formed, and long and short lower spacers 7 and 8 are disposed via an adhesive agent. A lower groove 9 is formed between the long and short lower spacers 7 and 8, and potassium ferricyanide 19 (19A, 19B) is placed in the groove 9. Fine crystal particles of the potassium ferricyanide 19 (19A, 19B) having a maximum crystal particle diameter of 100 µm or less are precipitated by subjecting an aqueous solution containing the potassium ferricyanide 19 (19A, 19B) to freezing, heating, or mixing with a poor solvent in accordance with the methods shown in Figs. 2 to 4.

A right part of the insulating sheet substrate 25 serves as an upper substrate 15A, and long and short upper spacers 12 and 13 are disposed on the substrate 15A via an adhesive agent. An upper groove 14 is formed between the long and short upper spacers 12 and 13, and an enzyme is placed in the groove 14. As one example of placing the enzyme, the method shown in Fig. 2 may be employed. After that, the one insulating sheet substrate 25 is folded into a laterally-faced U shape as shown in Fig. 5(B) to attach the short spacers 8 and 13 to each other as well as the long spacers 7 and 12 to each other via an adhesive agent 10. Thus, a reaction chamber 17 is formed by the opposed upper and lower grooves 9 and 14. In the reaction chamber 17, the potassium ferricyanide 19 (19A, 19B) and the enzyme is opposed with a gap being defined therebetween, so that activity of the enzyme is maintained.

Though this invention is described in detail and with reference to the specific embodiments in the foregoing, it is apparent to person skilled in the art that it is possible to add various modifications and alterations insofar as the modifications and alterations do not depart from the spirit and scope of this invention. This patent application is based on Japanese patent application filed on October 17, 2005 (Patent Application Number: 2005-302330), and contents thereof are incorporated herein by reference.

## Claims

1. A biosensor chip comprising:
upper and lower substrates,
at least two electrodes disposed on at least one of the upper and lower substrates, and
a reaction chamber for performing a chemical reaction, wherein
at least an enzyme and potassium ferricyanide are included as reagents to be placed in the reaction chamber;
a capacity of the potassium ferricyanide placed in the reaction chamber is V × 0.1 mg or more when a capacity of the reaction chamber is V µL; and
a maximum diameter of crystal particles of the potassium ferricyanide is 100 µm.

2. The biosensor chip according to claim 1, wherein
the maximum diameter of the crystal particles of the potassium ferricyanide may preferably be 50 µm or less.

3. The biosensor chip according to claim 1 or 2, wherein
the enzyme and the potassium ferricyanide are placed in the reaction chamber with a gap being defined therebetween.

4. The biosensor chip according to any one of claims 1 to 3, wherein
the upper and lower substrates are formed from one sheet, and
the one sheet is folded to form the upper and lower substrates.

5. A biosensor chip production method for producing a biosensor chip including upper and lower substrates, at least two electrodes disposed on at least one of the upper and lower substrates, and a reaction chamber for performing a chemical reaction, the method comprising:
a step of coating at least an enzyme and potassium ferricyanide in the reaction chamber, and
a step of freezing, heating, or mixing with a poor solvent the potassium ferricyanide in such a manner that a maximum diameter of crystal particles of the potassium ferricyanide becomes 100 µm or less and a capacity of the potassium ferricyanide placed in the reaction chamber becomes V × 0.1 mg or more when a capacity of the reaction chamber is V µL.

6. The biosensor production method according to claim 5, wherein
one sheet is folded to form the upper and lower substrates.
